# EUROPÄISCHE PATENTSCHRIFT

(11) **EP 2 149 346 B1**
(45) Veröffentlichungstag und Bekanntmachung des Hinweises auf die Patenterteilung: **25.07.2018**
(21) Anmeldenummer: 09161978.3
(22) Anmeldetag: 04.06.2009
(51) Int. Cl.: A61C 13/00, A61K 6/10

(54) **Verwendung von ausbrennbaren, leicht fräsbaren CAD Blöcken aus Schaumkunststoff**
Use of burnable, readily machineable CAD blocks made of foam plastic
Utilisation de blocs calcinables en mousse, facilement fraisables par commande numérique

(30) Priorität: 29.07.2008 DE 202008008805 U; 21.01.2009 DE 202009000043 U
(43) Veröffentlichungstag der Anmeldung: 03.02.2010
(73) Patentinhaber: Ivoclar Vivadent AG, 9494 Schaan (LI)
(72) Erfinder: Bürke, Harald, 6820, Frastanz (DE)
(74) Vertreter: Fitzner, Uwe

(56) Entgegenhaltungen:
- EP-A1- 1 925 268
- DE-A1- 2 165 280
- DE-A1- 2 342 948
- JP-A- H 105 928
- US-A- 4 854 368
- US-A1- 2002 028 854

## Beschreibung

Die vorliegende Erfindung betrifft die Verwendung eines besonders geeigneten Materials für vollständig ausbrennbare Modelle, welche über ein CAD/CAM-Verfahren hergestellt werden für die Herstellung von dentalen Restaurationen oder Restaurationsteilen mittels Presstechnik.

In der Press-Technologie von Gläsern und Glaskeramiken, einer von Ivoclar Vivadent AG, Schaan, Liechtenstein unter dem Namen Empress® eingeführten und seit über 20 Jahren in der Dentaltechnik etablierten Technologie, wird für eine Restauration (Inlay, Onlay, Krone, Brücke, Veneer, Abutment etc.) ein Modell üblicherweise in Wachs von Hand modelliert. Dieses Wachsteil wird in einer feuerfesten Gießmasse (Einbettmasse) eingebettet. Während des anschließenden Vorwärmprozesses erhärtet die Gießmasse und das Wachsteil brennt rückstandsfrei aus. In den verbleibenden Hohlraum wird dann die zähflüssig erweichte glaskeramische Masse unter Druck eingepresst. In einem analogen Verfahren kann auch Metall geschmolzen und in den verbleibenden Hohlraum gegossen werden.
Ebenso ist es möglich, dass nur Teile einer solchen Restauration, z.B. die ästhetische Verblendung, auf ein vorhandenes Gerüst aus Metallen, Legierungen, Glas- oder Oxidkeramiken aufgepresst werden. Dazu wird auf das Gerüst die vorgesehene Verblendung mit Wachs modelliert und eingebettet, anschließend wie vorstehend beschrieben das Wachs ausgebrannt und in den Hohlraum die Verblendung aus Glas bzw. einer Glaskeramik eingepresst.
In zunehmendem Maße werden Restaurationen am Computer modelliert (CAD) und maschinell hergestellt (CAM).
Ein verbreitetes Verfahren zur maschinellen Herstellung ist ein subtraktives Verfahren. Das gewünschte Werkstück wird aus einem massiven Block herausgefräst oder -geschliffen.

Diese Maschinen (z. B. Sirona InLab® oder KaVo Everest®) sind in der Lage, dentale Restaurationen direkt aus für den Schleifprozess geeigneten Keramiken (z.B. Zirkonoxid, Aluminiumoxid oder Glaskeramiken auf Basis von z.B. Leucit, Leucit-Apatit, Feldspat oder Lithiumsilicat) herauszuschleifen. Häufig möchte man jedoch das CAD/CAM-Verfahren mit der Presstechnik verbinden, weil man nicht-fräsbare, jedoch verpressbare Keramikmaterialien mit der Presstechnik oder Metalle mit der Gusstechnikverarbeiten will.
Manche Materialien sind für die Schleiftechnik nicht geeignet, weil sie entweder zu spröde sind und brechen würden, oder weil sie zu zäh sind und einen hohen Werkzeugverschleiß und eine lange Bearbeitungsdauer verursachen würden. Oder man möchte Teile der Restauration mit der CAD/CAM -Technik herstellen und andere Teile mit der Presstechnik, um die Vorteile der einen mit den Vorteilen der anderen Technik zu vereinen.
Die vorstehend beschrieben Verfahren zur Herstellung von Restaurationen bzw. Teilen davon mit den beiden Verfahren Heißpressen von Glaskeramiken oder Gläsern einerseits sowie CAD/CAM-Verfahren andererseits haben unabhängig voneinander ihre Vorteile aber auch Grenzen für die Anwendungsmöglichkeiten.
So hat die CAD/CAM-Technik u.a. den Vorteil, dass ein Zeitgewinn möglich ist; auch sind die Daten gespeichert, so dass eine Restauration im Bedarfsfall reproduziert werden kann. Die Presstechnik hat den Vorteil der höchsten Passgenauigkeit, auch können komplizierte Geometrien, z.B. Hinterschneidungen, hergestellt werden, welche schleiftechnisch zwar möglich, aber nicht einsetzbar sind.
Beispielsweise kann man auf ein Zirkonoxidgerüst oder auf ein Metallgerüst ein Glaskeramikmaterial im Empress-Verfahren aufpressen. Das Zirkonoxidgerüst wird üblicherweise über einen CAM-Prozess gefertigt, da es sich im Empress-Verfahren nicht verpressen lässt. Das Metallgerüst dagegen kann gegossen oder gefräst sein. Bei einem CAD/CAM-Verfahren liegen die Daten für die komplette Restauration dann im besten Fall schon vor, und es ist einfach, die gewünschte Beschichtung am Computer zu entwerfen und den zweiten Teil, die Beschichtung, maschinell zu fräsen.
Um nun die Materialien für die Presstechnik verwenden zu können, kann ein vollständig ausbrennbares Material, welches fräsbar ist, maschinell gefertigt werden. Dieses wird mit dem Gerüst temporär verbunden. Der Verbund wird nun in bekannter Weise in feuerfester Gießmasse eingebettet. Der ausbrennbare Teil, welcher im Beispiel dem Verblendteil der Restauration entspricht, verbrennt vollständig, während das Gerüst unverändert bleibt. Im anschließenden Pressprozess wird der entstandene Hohlraum mit Presskeramik gefüllt. Nach dem Ausbetten liegt eine Restauration aus einem hochfesten Gerüst und einer ästhetischen Beschichtung vor.

Für diesen Zweck wird z.B. von der Firma VITA ein spezieller Block angeboten. Der Block heißt entsprechend der bestimmungsgemäßen Verwendung CAD-Waxx for InLab®, weil Wachs für die Ausbrenntechnik in idealer Weise geeignet ist.
Wachs ist jedoch für die maschinelle Bearbeitung nur schlecht geeignet, da es zum einen die Schleifwerkzeuge verschmiert und zum anderen leicht bricht. Der CAD-Waxx Block ist daher aus einem massiven, vollständig ausbrennbaren Kunststoff (PMMA) gefertigt.
Dieses massive Kunststoffmaterial zeigt einige zum Teil gravierende Nachteile:
- Es kann nur sehr langsam bearbeitet werden, da es bei starker Wärmeentwicklung duktil wird und dann zum Verschmieren der Schleifwerkzeuge neigt.
- Der entstehende Schleifstaub verstopft schnell die Kühlwasserfilter.
- Nach dem Einbetten mit feuerfester Einbettmasse entsteht durch eine chemische Reaktion (Abbindereaktion der Einbettmasse) Wärme. Da die thermische Expansion des Kunststoffs höher ist als die thermische Expansion resultierend aus der Abbindereaktion der Einbettmasse, entstehen Druckspannungen auf die Einbettmasse. Diese Spannungen können zu Rissen in der Einbettmasse führen. Es können deshalb keine dickwandigen Objekte eingebettet werden.

Aus DE 21 65 280 A1 ist die Verwendung von Schaumstoffen als intergralem Teil von Pressformen bekannt. In diesem Dokument wird weder der Dentalbereich erwähnt, noch dass der Schaumstoff in der Presstechnik zur Herstellung von dentalen Restaurationen oder Restaurationsteilen eingesetzt werden kann.
Aus EP 1 925 268 A1 ist es bekannt, Kunststoffe in der Presstechnik als Modellierhilfe einzusetzen. Die Verwendung von Schaumkunststoffen geht jedoch nicht aus diesem Dokument hervor.
Aus DE 23 42 948 A1 ist die Verwendung von Schaumkunststoffen bei der Herstellung von Keramikhohlkörpern bekannt, wobei die Keramikhohlkörper als Wärmeträger, als Zuschlagstoffe für Baumaterialien, als Füllkörper für Austauschertürme oder Fraktionierkolonnen und als Katalysatorträger eingesetzt werden können. Ein Hinweis auf den Dentalbereich und dessen spezielle Anforderungen findet sich nicht. In der Entgegenhaltung JP 10-005928 wird für ein auszubrennendes Produkt beispielhaft auf Schaumkunststoffe, gleichberechtigt neben Wachsen und schmelzenden Kunststoffen usw. verwiesen. Die Akronyme CAD bzw. CAM werden im Rahmen dieser Erfindung verstanden, wie sie z.B. in Römpp Chemie Lexikon, 9. Auflage, Band 1, 1989, Seiten 541 und 565 dargestellt sind. Da die Terminologie CAD/CAM darüber hinaus fachnotorisch bekannt ist, sind dem Fachmann weitere bzw. genauere Ausgestaltungen hinlänglich bekannt.
Aufgabe der vorliegenden Erfindung ist es, einen Werkstoff bzw. ein Werkstück zur Verwendung in der Presstechnik, zur Verfügung zu stellen, der/das die vorgenannten Nachteile nicht mehr aufweist.
Diese Aufgabe wird durch die Verwendung eines Kunststoffblocks aus Schaumkunststoff in der Presstechnik gemäß Anspruch 1 gelöst.

Die vorgenannten Nachteile können überraschenderweise vermieden werden, wenn man an Stelle eines massiven Kunststoffes einen Schaumkunststoff verwendet. Schaumkunststoffe sind Werkstoffe mit einer zelligen Struktur, wobei man offenporige und geschlossenporige Schaumkunststoffe unterscheidet. Als besonders vorteilhaft haben sich zäh-harte (aus Polystyrol, Polymethylmethacrylaten, Styrol-Acrylnitril-Copolymeren, Polyethylen, Polypropylen oder Polyurethan) und spröd-harte (aus Polyurethan, Phenol-oder Harnstoff-Formaldehyd-Harze) Schaumkunststoffe herausgestellt.

Ganz besonders geeignet ist geschäumtes Polystyrol.
Insbesondere werden geschlossenporige Schaumkunststoffe mit einer Rohdichte von kleiner als 130 kg/m³ bevorzugt, ganz besonders bevorzugt sind Materialien mit einer Rohdichte von weniger als 80 kg/m³, sofern sie folgende Bedingungen erfüllen:
- Das Material ist formstabil. Es weist eine Maßhaltigkeit von besser als 0,2% auf. Maßhaltigkeit bedeutet, daß die Ist-Maße des Werkstücks nach der mechanischen Bearbeitung innerhalb einer Abweichung von 0,2% vom vorgegebenen Nennwert liegen, also die zulässige Maßabweichung vom Soll-Wert. Die Maßhaltigkeit ist ein Ausdruck für die geometrische Beständigkeit des Werkstoffs gegen Dehnung und Schrumpfung während der mechanischen Bearbeitung.
- Das Material weist im Temperaturbereich von 20°C bis 40°C eine hohe Steifigkeit auf, d.h. es hat ein E-Modul von >5MPa.
   Das Material weist eine Druckfestigkeit von >100kPa auf.
- Das Material ist wasserbeständig. Es nimmt bei einer Lagerung von 24h bei Raumtemperatur in Wasser nur höchstens 0,2% Wasser auf, d.h. es resultiert eine Zunahme von 0,2 Gew.-% bezogen auf das Ausgangsmaterial.
- Um eine genügende Oberflächenqualität zu erzielen, sind besonders mikro-, fein- oder mittelzellige Qualitäten zu verwenden. Dabei bedeutet mikrozellig einen mittleren Porendurchmesser von kleiner 0,3 mm, feinzellig von 0,3 bis 0,5 mm, und mittelzellig von 0,5 bis 3 mm. Erfindungsgemäß sind mittlere Porendurchmesser von kleiner als 1 mm bevorzugt, kleiner als 0,5 mm besonders bevorzugt und zwischen 0,05 und 0,15 mm ganz besonders bevorzugt, jeweils bestimmbar mittels Lichtmikroskop.
- Das Material ist weiterhin bei Temperatur zwischen 750°C und 900°C unter Luftatmosphäre rückstandsfrei ausbrennbar.

Beispiele für geeignete Schaumkunststoffe sind Neopor® und Styropor® von BASF bzw. IsoBouw, geschäumte PE- (Plastazote®), PP- (Propozote®) und PA- (Zotek®)Typen von W. Köpp oder auch Jackocell® R von Jackon.

Ein solcher Block aus Schaumkunststoff zeigt folgende Vorteile:
- Die Bearbeitungsgeschwindigkeit für einen Block aus XPS (extrusionsgeschäumtes Polystyrol) ist sehr hoch (15 Minuten für eine viergliedrige Musterbrücke), bis zu viermal schneller als beim bestehenden Material (ca. eine Stunde für die gleiche Musterbrücke mit einem CAD-Waxx von VITA) und nur durch die maximale Geschwindigkeit der Bearbeitungsmaschine limitiert.
- Die Wärmeentwicklung ist vernachlässigbar klein, die Schleifwerkzeuge setzen sich nicht zu und sie unterliegen praktisch keinerlei Verschleiß.
- Es entsteht nur wenig Schleifstaub. Die Reinigungsintervalle werden wesentlich länger.
- Während der Abbindereaktion der Einbettmasse entsteht durch die Wärmeausdehnung der Schaumkunststoffe nur ein geringer Druck auf die Einbettmasse. Zwar zeigen auch Schaumkunststoffe die für Kunststoffe übliche hohe Wärmeausdehnung, jedoch wird die Luft in den geschlossenen Poren komprimiert, wodurch lediglich unkritische Druckspannungen entstehen können. Die Abmessungen der eingebetteten Objekte sind daher nicht limitiert.
- Beim Ausbrennen entstehen weniger Verbrennungsprodukte, was unter Umweltgesichtspunkten oder in Bezug auf die Abgasreinigung vorteilhaft ist, außerdem wird das Risiko von Ablagerungen aus entstehendem Kohlenstoff (z.B. Ruß) in Bereichen mit kleinen Geometrien deutlich reduziert.
Die Herstellung solcher Blöcke ist sehr preiswert, zumal die Halter zur Befestigung in den Bearbeitungsmaschinen zweckmäßigerweise ebenfalls aus preiswerten Kunststoffen gefertigt werden können. Während die Blöcke vorteilhafterweise aus Polystyrol, Polyurethan, Phenol-Formaldehydharze, Polymethylmethacrylat, Polyethylen, Polypropylen oder Styrol-Acrylnitril-Copolymer hergestellt werden, werden die Halter zum Einspannen der Blöcke in die CAD/CAM-Maschinen bevorzugt angeklebt und können beispielsweise aus einem (faserverstärkten) Polyamid, Polystyrol, Polypropylen oder HD-Polyethylen bestehen.

Solche Kunststoffblöcke lassen sich demgemäß hervorragend für die Herstellung von Modellen für die Presstechnik von dentalen Restaurationen oder Restaurationsteilen verwenden.
Im Rahmen dieser Verwendung werden die Kunststoffblöcke insbesondere dergestalt eingesetzt, daß der Hohlraum für das Verpressen durch Ausbrennen von Teilen der Kunststoffblöcke gebildet wird.
Besonders bevorzugt werden die erfindungsgemäßen Kunststoffblöcke demgemäß als Modellwerkstoff für Presstechniken, wie insbesondere das Empress® Verfahren, verwendet.

### Beispiele:

### Beispiel 1:

Aus herkömmlichem Styropor-Verpackungsmaterial einer mittleren Porengröße von 0,5 mm wurden Blöcke der Abmessungen von etwa 35x14x14 mm³ hergestellt, an einer Stirnseite wurde ein Kunststoffhalter durch Kleben befestigt.

Aus so hergestellten Blöcken wurden Modelle für Kronen und Inlays gefräst, wie gewohnt eingebettet und nach dem Ausbrennen durch Einpressen eines Glases oder einer Glaskeramik nach dem Empress-Verfahren Restaurationen hergestellt.

Es konnte mit diesen Modellen eine gute Passgenauigkeit realisiert werden, jedoch war die Oberflächenqualität der gepressten Restauration gegenüber einem Vollkunststoff und erst recht gegenüber einer Wachsmodellation geringer. Da jedoch die Restaurationsteile, die nach dem Pressverfahren hergestellt werde, eine abschließende ästhetische Beschichtung erfahren, spielt die höhere Rauhigkeit keine negative Rolle.

### Beispiel 2:

Beispiel 1 wurde unter Verwendung von Jackocell® R M1 mit einer mittleren Porengröße von etwa 0,1 mm als Ausgangsmaterial wiederholt.
Es konnte mit diesem Beispiel gezeigt werden, daß die Verwendung von Schaumkunststoffen mit einer kleineren Porengröße eine deutlich verbesserte Oberflächengüte der gepressten Restaurationsteile ermöglicht.

Nach dem Ausbetten des Restaurationsteile erfolgte gemäß Stand der Technik die Reinigung durch Sandstrahlen, Polieren und Verblenden mit einer Glasurmasse.

### Vergleichsbeispiel:

Aus einem CAD-WAXX-Block der Größe CW-40 (Fa. Vita, Abmessung 14x15x40 mm) wurde mit der Bearbeitungsmaschine InLab MCXL (Fa. Sirona) ein viergliedriges Brückengerüst geschliffen. Die Schleifzeit betrug 64 Minuten.

### Beispiel 3:

Aus einem handelsüblichen Polystyrolschaumstoff mit einem mittleren Porendurchmesser von 0,2 mm wurden Blöcke der Abmessungen von 14x15x40 mm hergestellt, an einer Stirnseite wurde ein Kunststoffhalter mit Heißwachs befestigt. Das gleiche viergliedrige Brückengerüst wie im Vergleichsbeispiel wurde geschliffen. Die Schleifzeit betrug nur 18 Minuten. Durch Optimierung der Schleifstrategie wäre eine weitere Zeiteinsparung möglich.

### Beispiel 4:

Aus einem mikrozelligen (<0,3mm) extrudierten Polystyrolschaumstoff (XPS) wurden Blöcke der Abmessungen von 14x15x40 mm hergestellt und mit Heißwachs auf Halter geklebt. Aus so hergestellten Blöcken wurden Kronen, Inlays und Brückengerüste geschliffen. Die Schleifzeiten waren jeweils sehr kurz und betrugen nur ca. 20 bis 30% der Schleifzeiten, die für die gleichen Modelle gebraucht wurden, wenn diese aus CAD-WAXX der Firma Vita gefertigt wurden. Die Passgenauigkeit der Schaumstoffmodelle wurde an Modellstümpfen überprüft.

Anschließend wurden die Schaumstoffmodelle wie üblich eingebettet (Einbettmasse PressVest Speed, Fa. Ivoclar) und im Vorwärmofen bei einer Temperatur von 850°C ausgebrannt. Anschließend wurde eine Presskeramik (e.max Press LT) bei einer Temperatur von 920°C nach dem Empress-Verfahren verpresst und die Passgenauigkeit der Objekte auf dem Modellstumpf überprüft.

Es wurden sehr gute Passgenauigkeiten erhalten.

## Patentansprüche

1. Verwendung von Kunststoffblöcken für die Herstellung von dentalen Restaurationen oder Restaurationsteilen mittels Presstechnik, wobei der Kunststoffblock aus einem Schaumkunststoff besteht und wobei der Schaumkunststoff
- eine Dichte von kleiner als 130 kg/m³ aufweist,
- formstabil ist,
- im Temperaturbereich von 20°C bis 40°C ein Modul von > 5 MPa aufweist,
und eine Druckfestigkeit von > 100 kPa aufweist
- wasserbeständig ist,
- einen mittleren Porendurchmesser von zwischen 0.05 und 0,15 mm aufweist,
- rückstandsfrei ausbrennbar ist.

2. Verwendung nach Anspruch 1, **dadurch gekennzeichnet, dass** der Schaumkunststoff ausgewählt wird aus der Gruppe bestehend aus offenporigen und geschlossenporigen Schaumkunststoffen.

3. Verwendung nach Anspruch 1 oder 2, **dadurch gekennzeichnet, dass** der Schaumkunststoff ausgewählt wird aus der Gruppe bestehend aus Polystyrol, Polymethylmethacrylat, Polyurethan, Phenol- oder Harnstoff-Formaldehydharzen, Polyethylen, Polypropylen und Styrol-Acrylnitril-Copolymeren.

4. Verwendung nach einem der Ansprüche 1 bis 3, **dadurch gekennzeichnet, dass** an dem Kunststoffblock ein Halter angebracht ist.

5. Verwendung nach einem der Ansprüche 1 bis 4, **dadurch gekennzeichnet, dass** durch Ausbrennen von Teilen der Kunststoffblöcke ein Hohlraum für das Verpressen gebildet wird.

## Claims

1. Use of plastic blocks for the preparation of dental restaurations or parts of restaurations by means of molding technique, wherein the plastic block consists of a foam plastic and the foam plastic
- has a density of less than 130kg/m³
- is dimensionably stable,
- in the temperature range of from 20C° to 40C° has a module of
> than 5MPa and
a compressive strength of > than 100 kPa,
- is water resistant,
- has an average pore diameter of from 0.05 and 0.15 mm,
- is residue-free combustible.

2. Use according to claim 1, **characterized in that** the foam plastic is selected from the group consisting of open-pore and closed-pore foam plastics.

3. Use according to claim 1 or 2, **characterized in that** the foam plastic is selected from the group consisting of polystyrene, polymethyl methacrylate, polyurethane, phenol- or urea-formaldehyde resins, polyethylene, polypropylene, and styrene-acrylonitrile copolymers.

4. Use according to any of the claims 1 to 3, **characterized in that** a holder is fastened at the plastic block.

5. Use according to any of the claims 1 to 4, **characterized in that** by burning out parts of the plastic blocks, a cavity is formed by pressing.

## Revendications

1. Utilisation d'un bloc en matière plastique pour la préparation des restaurations dentales ou des parts de restauration par la technique de pressage, où le bloc en matière plastique consiste d'une matière plastique de mousse et la matière plastique de mousse
- a une densité de moins de 130kg/m³
- est indéformable,
- a dans le secteur de température de 20C° à 40C° une module de >5MPa et
une résistance à la compression de > 100 kPa,
- est résistante à l'eau,
- a un diamètre de pore moyen d'entre 0.05 et 0.15 mm,
- est combustible exempte de résidus.

2. Utilisation selon la revendication 1, **caractérisée en ce que** la matière plastique de mousse est choisie du groupe composé de matières plastiques en mousse des pores ouvertes ou fermées .

3. Utilisation selon la revendication 1 ou 2, **caractérisée en ce que** la matière plastique en mousse est choisie du groupe composé de polystyrène, méthacryle de polyméthyle, polyuréthane, des résines de phénole- ou urée- formaldéhyde, polyéthylène, polypropylène, et styrène-acrylonitrile copolymères.

4. Utilisation selon l'une quelconque des revendications 1 à 3, **caractérisée en ce qu'**un support est fixé au bloc de matière plastique.

5. Utilisation selon l'une quelconque des revendications 1 à 4, **caractérisée en ce que** par combustion de parts des blocs en matière plastique une cavité est formée pour le procédé de pressage.
